# EUROPEAN PATENT APPLICATION

(11) **EP 4 631 512 A1**
(43) Date of publication of application: **15.10.2025**
(21) Application number: 24841675.2
(22) Date of filing: 25.10.2024
(51) Int. Cl.: A61K 36/15, A23K 10/12, A23K 10/30, A23K 20/142, A23K 50/42, A61K 9/36, A61K 31/36, A61K 31/198, A61K 31/352, A61K 33/06, A61K 33/14, A61K 33/24, A61K 33/32, A61K 36/185, A61K 47/26, A61K 47/36, A61P 9/00, A61P 43/00, A61K 129/00, A61K 131/00

(54) **ATRIAL NATRIURETIC PEPTIDE DEPRESSOR THERAPEUTIC AGENT AND PROPHYLACTIC AGENT AND METHOD FOR PRODUCING SAME**

(30) Priority: 15.02.2024 JP 2024021170
(71) Applicant: Scarecrow Inc., Shibuya-ku Tokyo 150-0045 (JP)
(72) Inventor: OHKAWA Hiroshi, Tokyo 150-0045 (JP); KOIE Hiroshi, Fujisawa-shi, Kanagawa 251-0053 (JP)
(74) Representative: TBK
(86) International application number: PCT/JP2024/038138
(87) International publication number: WO 2025/173317

(57) **Abstract**

[Solution]

By using a naturally occurring substance, heart disease such as a myxomatous mitral valve disease is more effectively suppressed without impairing the health of small animals.

[Effects]

An agent contains, as main ingredients, 5 to 9 wt% of a pine bark dry extract derived from bark of pine trees grown on the southwestern coast of France under exposure to intense ultraviolet light and containing proanthocyanidin, and 5 to 9 wt% of a fermented sesame dry extract containing sesamin, one of the antioxidant lignans, the fermented sesame dry extract mainly containing sesame as a main raw material, and contains 79 to 87 wt% of an excipient such as starch or lactose, as a raw material, which makes a powder uniform, solidifies a tablet, and prevents quality deterioration due to moisture absorption or the like, such as starch or lactose.

## Description

### Technical Field

The present invention relates to an atrial natriuretic peptide lowering, therapeutic, and prophylactic agent for a small animal suffering from mitral regurgitation, particularly a dog, and a method for producing the same. The abbreviation for atrial natriuretic peptide is ANP.

### Background Art

Recently, in pet breeding of small animals such as dogs and cats, due to improvement of nutritional value of pet food and excessive feeding, the small animals become obese easily. When a small animal becomes obese, a risk of heart disease increases as in humans. One type of heart disease is mitral regurgitation.

### <Overview of Mitral Regurgitation>

The heart is an important organ that performs a pump function of circulating blood to the whole body and the lung. Blood collected from the whole body into the vena cava takes in fresh oxygen in the lung from the right atrium through the right ventricle and is sent again from the aorta to the whole body from the left atrium through the left ventricle. When the small animal is healthy, the blood is always carried through this route.

The mitral valve in the heart is a valve that partitions the left atrium and the left ventricle, and when an abnormality occurs in the mitral valve, the valve is incompletely closed, and blood flows backward from the left ventricle to the left atrium. The "myxomatous mitral valve disease" is the most common heart disease in dogs, and the mitral valve becomes thicker, shorter, and malformed as the disease progresses, resulting in backflow of blood. This heart disease is referred to as "mitral valve insufficiency" or simply "mitral regurgitation".

When the reflux blood volume increases, the blood stasis in the heart pushes and spreads the heart, and cardiomegaly progresses. To some extent, the increased blood volume can be sufficiently delivered to the whole body by compensatory increase in cardiac function. However, progression to severe cardiomegaly cannot be compensated for and breaks down. That is, "pulmonary edema" in which the blood stagnated in the left atrium leaks into the lung to impair respiration is developed. Once pulmonary edema occurs, respiratory failure can progress rapidly, and delay in treatment can lead to death.

### <Symptoms of Mitral Regurgitation>

There are few early symptoms of mitral regurgitation, and in many cases, the symptoms are just a little fatigue and reduced play time. As the symptoms progress gradually, a patient becomes easily fatigued when exercising or excited, and exhibits the cyanosis phenomenon in which the tongue turns purple. Symptoms such as increased sleeping time are observed. Increased cough is also a bad sign. As the disease state further progresses, the patient does not move much and gets tired quickly, and the cough does not stop with a slight stimulus, leading to cyanosis or syncope.

When mitral regurgitation becomes severe and pulmonary edema develops, the patient has symptoms such as restlessness due to difficulty in breathing, breathing with a shrug while sitting down without lying down, persistent breathless cyanosis, and gasping for breath by stretching their neck. Eventually, the patient has symptoms such as exhalation of a blood-tinged pink liquid with cough, lying down with lethargic and unresponsive behavior due to cerebral hypoxia, and death occurs from respiratory failure.

### <Diagnosis of Mitral Regurgitation>

A cardiac murmur can be heard when regurgitation occurs in the mitral valve. However, since diagnosis of heart disease and evaluation of severity cannot be performed only by auscultation, examination for objectively observing the shape, size, and function of the heart is often performed subsequently. X-ray inspection, ultrasonic inspection, blood pressure measurement, electrocardiography, and the like are performed. Furthermore, it is also important to perform a blood test or a urine test in order to grasp whether heart disease is associated with a problem of another organ.

### <Stage of Mitral Regurgitation>

A stage of mitral regurgitation is generally divided into the following five stages and described. This is a classification by the American College of Veterinary Internal Medicine (ACVIM).

### (1) Stage A

Stage A is a stage where there is no abnormality in the heart at the present time.

Examples of dog breeds having a high risk of developing heart failure in the future include Cavalier King Charles Spaniel and Chihuahua.

### (2) Stage B1

Stage B1 is a stage where a cardiac murmur, valve degeneration, and mitral regurgitation begin to appear. However, this is a stage where cardiomegaly is not recognized.

### (3) Stage B2

Stage B2 is a stage where a cardiac murmur, valve degeneration, and mitral regurgitation are observed, and cardiomegaly is observed.

### (4) Stage C

Stage C is a stage where there are symptoms such as cough and shortness of breath and pulmonary edema has been treated in the past.

### (5) Stage D

Stage D is a stage where a therapeutic response has deteriorated despite all medical treatments.

This stage classification is very famous and is referenced by many veterinarians who provide cardiology care. Although it is simple and easy to understand, patients with different levels of severity are included in the same stage, or the rate of progression varies greatly for each individual patient, and thus, it is essential to grasp the disease state frequently by periodic medical examinations.

### <Treatment of Mitral Regurgitation>

In the treatment of mitral regurgitation, a recommended treatment directly depends on the severity. However, as described above, since the disease state of each patient varies even when the stage is the same, the actual treatment becomes a little more complicated.

### (1) Stage A

Pharmacotherapy or dietary therapy is not recommended.

### (2) Stage B1

Pharmacotherapy or dietary therapy is not recommended. It is recommended to perform a cardiac ultrasound examination and an X-ray examination once every 6 to 12 months.

### (3) Stage B2

It is strongly recommended to use a type of cardiotonic called pimobendan. Dietary therapy is recommended. Depending on the disease state, the use of antihypertensive agents, β-blockers, aldactone (weak diuretic), and the like is considered.

### (4) Stage C

In the acute phase, that is, pulmonary edema, intensive care with potent diuretics (furosemide), pimobendan, sedatives, antihypertensive agents, and oxygen therapy is strongly recommended under hospitalization. When dyspnea does not improve and progresses, respiratory assistance with a ventilator under anesthesia is considered for lifesaving.

In the chronic phase, that is, after withdrawal of pulmonary edema, continuous administration of diuretics, pimobendan, and antihypertensive agents, and frequent reevaluation of renal values and cardiac tests are strongly recommended. Dietary therapy or a record of health, such as a weight, at home is also recommended.

### (5) Stage D

Even when a high dose of a diuretic, pimobendan, an antihypertensive agent, or the like is used, improvement of the disease state is not observed and the disease state is unstable, and a treatment according to the situation is required each time. Cardiac surgery by a specialized cardiac surgery team can be a breakthrough.

A technique for evaluating the risk of disease onset in dogs suspected of having mitral regurgitation has been proposed. For example, Patent Literature 1 proposes a "method for evaluating a risk of disease onset in dogs", the method including a step of measuring body fat percentages of dogs and classifying the body fat percentages into a case where the body fat percentage is 35% or more and a case where the body fat percentage is less than 35%, and a step of classifying ages of the dogs to be measured into 5 years or older and younger than 5 years old, in which in a case where the age of the dog is 5 years or older and the body fat percentage is 35% or more, it is determined that the risk of onset of mitral regurgitation and/or tricuspid regurgitation is high.

### Citation List

### Patent Literature

Patent Literature 1: JP 2011-97868 A

### Summary of Invention

### Technical Problem

However, Patent Literature 1 is intended to help prevent disease onset in dogs by a simple operation of simply measuring the body fat percentage and by controlling the body fat percentage to be less than a specific value by dietary management. It only greatly contributes to the health management of the dogs. It did not improve the health of affected small animals.

In the conventional treatment method, it is difficult to find an abnormality in the heart at an early stage such as Stage A or Stage B1 because the abnormality hardly appears in the heart. In addition, in the treatment of mitral regurgitation, drug therapy or dietary therapy is not recommended at this early stage, and thus, there is a problem that it is likely to be late when symptoms greatly change.

The inventors of the present invention have intensively studied in view of such a situation. The present inventors have paid attention to the fact that an extract of bark has a strong antioxidant action or antiinflammatory action. Proanthocyanidin contained in a pine bark extract has an effect of suppressing oxidation of bad (LDL) cholesterol and preventing a lifestyle disease. In addition, there is also an effect of promoting blood flow. Therefore, the inventors of the present invention have considered that heart disease such as myxomatous mitral valve disease often progresses relatively slowly, and when the heart disease is noticed without subjective symptoms, the heart disease is late stage or the animal suddenly dies on a certain day. The present inventors have considered that even when a naturally occurring substance is taken at all times like a supplement, there is no possibility that health is impaired, and rapid worsening of symptoms can be prevented.

Furthermore, the present inventors have also paid attention to the fact that sesame is highly effective as a functional food material. Sesame has long been eaten as a food having a high nutritional value, and in particular, sesamin has attracted attention as an antioxidant food material derived from sesame. Furthermore, the present inventors have paid attention to the fact that a fermented product of sesame remarkably enhances the antioxidative property and immunostimulatory action inherent in sesame, and have considered that the fermented product of sesame can also contribute to improving blood pressure.

When a dog suffers from mitral regurgitation, the blood volume pumped out of the heart is reduced. A living body attempts to maintain the systemic blood pressure by contracting peripheral blood vessels in order to prevent a drop in blood pressure by homeostasis. Therefore, the blood volume returning to the heart is maintained. However, at the same time, blood circulation failure of peripheral tissues and a volume load on the heart occur. The volume load of the heart stretches the right atrial muscle as well as the left atrial muscle. The "pulmonary edema" is caused when left atrial blood pressure rises too much due to excessive left atrial volume load. In such a situation where the atrial muscle is extended, atrial natriuretic peptide (ANP) is excessively secreted from the atrial muscle.

The inventors of the present invention have focused on the fact that the pine bark extract and the fermented product of sesame described above have an action of relaxing peripheral blood vessels. By the action of relaxing the peripheral blood vessels, the blood volume returning to the heart is reduced, and the volume load on the atrium is reduced. This action reduces the amount of ANP secreted. It was considered that the pine bark extract and the fermented product of sesame have an effect of suppressing onset of pulmonary edema and the like by finally reducing the left atrial volume load.

The inventors of the present invention have focused on the fact that the pine bark extract and the fermented product of sesame are effective in improving mitral regurgitation in dogs, but a blending ratio thereof has been unknown. It was considered that there was an optimum blending ratio capable of reducing the left atrial volume load without increasing the burden on the heart of each dog.

The present invention has been made to solve such problems. That is, an object of the present invention is to provide an atrial natriuretic peptide lowering, therapeutic, and prophylactic agent that can more effectively suppress heart disease such as myxomatous mitral valve disease without impairing the health of small animals by using a naturally occurring substance, and a method for producing the same.

### Solution to Problem

An atrial natriuretic peptide lowering, therapeutic, and prophylactic agent of the present invention is an atrial natriuretic peptide lowering, therapeutic, and prophylactic agent for an animal suffering from mitral regurgitation, in which the atrial natriuretic peptide lowering, therapeutic, and prophylactic agent is obtained by:
extracting bark of pine trees at 10 to 50°C using 30 wt% or more of an extract to produce a pine bark extract, the pine trees having been grown on the southwestern coast of France under exposure to intense ultraviolet light;
adding 30 wt% or more of an amino acid to sesame seeds, inoculating one or more bacteria selected from lactic acid bacteria, and performing fermentation at 10 to 50°C to produce a fermented sesame dry extract; and
blending the fermented sesame dry extract with the concentrated pine bark dry extract so that a weight mixing ratio of the pine bark dry extract and the fermented sesame dry extract is 0.5 to 2.0 of a weight ratio of the fermented sesame dry extract with respect to 1 of a weight ratio of the pine bark dry extract.

The atrial natriuretic peptide lowering, therapeutic, and prophylactic agent further contains 0.5 to 0.9 wt% of glutathione yeast containing glutamic acid, cysteine, and glycine.

The fermented sesame dry extract is an extract containing organic selenium in a weight ratio of 75 ppm or more.

The atrial natriuretic peptide lowering, therapeutic, and prophylactic agent can be blended with potassium, magnesium, zinc, manganese, or copper as an essential mineral supplement.

Antioxidant lignans including sesamin and sesamolin can be blended with the atrial natriuretic peptide lowering, therapeutic, and prophylactic agent.

It is desirable that the atrial natriuretic peptide lowering, therapeutic, and prophylactic agent is obtained by coating a tablet solidified to a certain hardness by pressure with polysaccharides including starch, glycogen, and cellulose and coating the outside of the table with water-soluble monosaccharides and disaccharides including starch and dextrin.

The atrial natriuretic peptide lowering, therapeutic, and prophylactic agent can be filled in a capsule having a double structure in which an inner coating 12 is formed of starch and an outer coating 11 is formed of a gelatinous material that is more water-soluble than the inner coating 12.

A method for producing an atrial natriuretic peptide lowering, therapeutic, and prophylactic agent of the present invention includes:
extracting bark of pine trees at 10 to 50°C using 30 wt% or more of an extract to produce a pine bark extract, the pine trees having been grown on the southwestern coast of France under exposure to intense ultraviolet light;
concentrating the pine bark extract to a moisture content of 5 to 20%;
adding 30 wt% or more of an amino acid to sesame seeds, inoculating one or more bacteria selected from lactic acid bacteria, and performing fermentation at 10 to 50°C to produce a fermented sesame dry extract; and
blending the fermented sesame dry extract with the concentrated pine bark dry extract so that a weight mixing ratio of the pine bark dry extract and the fermented sesame dry extract is 0.5 to 2.0 of a weight ratio of the fermented sesame dry extract with respect to 1 of a weight ratio of the pine bark dry extract.

### Advantageous Effects of Invention

The atrial natriuretic peptide lowering, therapeutic, and prophylactic agent of the present invention can reduce a left atrial volume load without increasing a burden on the heart of the dog because the naturally occurring substance, the pine bark dry extract, and the fermented sesame dry extract containing sesame seeds as a main material are in optimal blending ratios without reducing the function of each of the pine bark dry extract, the fermented sesame dry extract, and the glutathione yeast. It is possible to prevent the symptoms from worsening rapidly without impairing the health of the small animal.

The glutathione yeast has an antioxidant action of glutathione and can contribute to improving blood pressure.

Proanthocyanidin contained in the pine bark dry extract can suppress oxidation of bad (LDL) cholesterol and prevent a lifestyle disease. The antioxidative property and immunostimulatory action inherent in sesame can contribute to improving blood pressure.

By forming the atrial natriuretic peptide lowering, therapeutic, and prophylactic agent into a sugar-coated tablet or double capsule, it is possible to prevent a drug from adhering to the palate or adhering to the throat or the inner wall of the esophagus. It is possible to administer the drug to small animals without worrying about a taste such as odor or bitterness.

### Brief Description of Drawings

Fig. 1 is a table showing blending ratios in an atrial natriuretic peptide lowering, therapeutic, and prophylactic agent of the present invention.
Fig. 2 is an enlarged cross-sectional view showing a sugar-coated tablet-type atrial natriuretic peptide lowering, therapeutic, and prophylactic agent in which a double sugar coating is formed.
Fig. 3 is an enlarged cross-sectional view showing an atrial natriuretic peptide lowering, therapeutic, and prophylactic agent filled in a double capsule.
Fig. 4 is a flowchart showing a method for producing an atrial natriuretic peptide lowering, therapeutic, and prophylactic agent of the present invention in Example 2.
Fig. 5 is a flowchart showing a process for producing a pine bark dry extract in detail.
Fig. 6 is a flowchart showing a process for producing a fermented sesame dry extract in detail.
Fig. 7 is a graph showing changes in ANP value before and after the atrial natriuretic peptide lowering, therapeutic, and prophylactic agent of the present invention is given to animals (dogs) suffering from mitral regurgitation (MR).
Fig. 8 is a graph showing changes in symptoms in each stage.
Fig. 9 is a graph showing a severity stage distribution.
Fig. 10 is a graph showing improvement of clinical symptoms when the atrial natriuretic peptide lowering, therapeutic, and prophylactic agent is administered to 9 dogs in Group A.
Fig. 11 is a graph showing improvement of clinical symptoms by administration of the atrial natriuretic peptide lowering, therapeutic, and prophylactic agent of the present invention in 9 cases of Group A.
Fig. 12 is a graph showing improvement of clinical symptoms by administration of the atrial natriuretic peptide lowering, therapeutic, and prophylactic agent of the present invention in 12 cases of Group B.
Fig. 13 is an image showing a comparison of X-ray images of the chest of the dog.
Fig. 14 is an image showing a comparison of X-ray images of the chest of the dog captured from another angle.
Fig. 15 is an image showing a comparison of ultrasonography (echography) for a right parasternal four-chamber cross section.
Fig. 16 is an image showing a comparison of ultrasonography (echography) for a right parasternal short-axis four-chamber cross section.
Fig. 17 shows a comparison of color jet areas, and is an image showing a comparison of ultrasonography (echography) for a left ventricular inflow blood velocity waveform.
Fig. 18 shows the examination of cardiac motion of a dog having symptoms of bradycardia and tachycardia syndrome.
Fig. 19 is an explanatory view showing improvement of clinical symptoms when the atrial natriuretic peptide lowering, therapeutic, and prophylactic agent of the present invention is administered to a dog having symptoms of bradycardia and tachycardia syndrome.
Fig. 20 is an image showing a comparison of X-ray images when the atrial natriuretic peptide lowering, therapeutic, and prophylactic agent of the present invention is administered to a dog having symptoms of a sick sinus syndrome.
Fig. 21 is an image showing a comparison of X-ray images when the atrial natriuretic peptide lowering, therapeutic, and prophylactic agent of the present invention is administered to a dog having symptoms of a sick sinus syndrome.

### Description of Embodiments

Raw materials of an atrial natriuretic peptide lowering, therapeutic, and prophylactic agent of the present invention mainly contain two types of extracts of pine bark and an extract obtained by fermenting sesame. The pine bark extract is a substance containing flavonoid called proanthocyanidin. The fermented product of sesame is a substance containing sesamin of an antioxidant lignan.

### [Example 1]

### <Pine Bark Dry Extract>

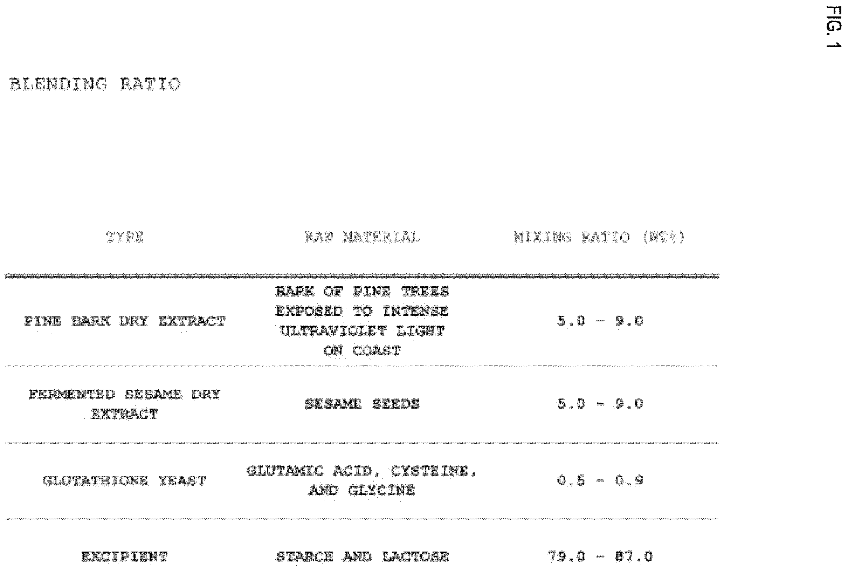

Fig. 1 is a table showing types, raw materials, and blending ratios in an atrial natriuretic peptide lowering, therapeutic, and prophylactic agent of the present invention.

The atrial natriuretic peptide lowering, therapeutic, and prophylactic agent of the present invention of Example 1 is a lowering agent, a therapeutic agent, and a prophylactic agent containing, as main ingredients, a pine bark dry extract containing proanthocyanidin and a fermented sesame dry extract containing sesamin of an antioxidant lignan mainly containing sesame as a main raw material. A mixing ratio of the pine bark dry extract and the fermented sesame dry extract is 0.5 to 2.0 of a weight ratio of the fermented sesame dry extract with respect to 1 of a weight ratio of the pine bark dry extract. The proanthocyanidin is a polyphenol having a structure in which catechin molecules are linked. It is an ingredient that functions as an antioxidant.

The first main ingredient of the atrial natriuretic peptide lowering, therapeutic, and prophylactic agent of the present invention, the "pine bark dry extract", contains, as an ingredient, an extract derived from natural plants taken from coastal pine bark growing on the southwestern coast of France. This French coastal pine bark extract is an ingredient extracted from bark of pine trees growing on the southwestern coast of France exposed to intense ultraviolet light. Since this French coastal land is in fine weather for 320 days or longer a year and thus exposed to significantly intense ultraviolet light, the pine trees growing on this land have developed to store a lot of antioxidants to protect themselves.

The proanthocyanidin contained in the pine bark dry extract, which is the main ingredient of the present invention, has an excellent antioxidant power that enhances the antioxidant power in the body. The proanthocyanidin has the function of increasing nitric oxide and improving the blood flow reinforcing the circulatory system. The proanthocyanidin has a binding function with collagen and elastin for repairing and strengthening the blood vessel wall. The proanthocyanidin has an inhibitory action on nuclear factor-kappa B (NF-kB) and a potent antiinflammatory action. NF-kB is a modulator of the inflammatory response acute phase, and is a factor required for effective immune defense and elimination of transformed cells. The proanthocyanidin has a function of suppressing an increase in blood glucose level after a meal and stabilizing the blood glucose level by suppressing α-glucosidase. The α-glucosidase has a function of assisting an action (hydrolysis) in which water reacts with and decomposes a portion where sugars are bonded to each other.

The heart disease risk reducing effect of the proanthocyanidin contained in the pine bark dry extract has a blood flow improving function. First, there is an effect of suppressing platelet aggregation. For humans, there are effects on platelet aggregation caused by smoking and effects on economy class syndrome. The proanthocyanidin also has a function of dilating blood vessels. The proanthocyanidin has a function of decreasing the production of endothelin-1, which is a vasoconstrictor substance, and promoting the production of prostacyclin, which is a vasodilator.

The proanthocyanidin contained in the pine bark dry extract has a heart disease risk reducing effect. Vascular endothelial cell-derived vascular regulators include the following. Endothelin is a potent vasoconstricting peptide derived from vascular endothelial cells and is increased in production during heart failure. Prostacyclin PGI₂ has an action of relaxing blood vessels and suppressing platelet aggregation. Nitric oxide (NO) is a representative of vascular relaxation factors derived from vascular endothelial cells.

### <Fermented Sesame Dry Extract>

The "fermented sesame dry extract" contains sesamin, which is an antioxidant lignan unique to sesame. Sesame has been eaten as a highly nutritive food since a long time ago. In recent years, its functionality has been studied, and a sesame lignan, which is a type of phenylpropanoid, has been used in many supplements as a functional food material. In particular, sesamin is attracting attention as an antioxidant food material derived from sesame. Antioxidation and immunity are important elements of recent functional foods, and they can improve a so-called pre-illness state, which is the root cause of all diseases. In the present invention, the functionality of a conventional sesame fermented product is enhanced, and γ-aminobutyric acid is efficiently produced by lactic acid fermentation in addition to the blood pressure suppressing effect inherent in sesame, thereby enhancing the blood pressure increase suppressing effect.

In the fermented sesame dry extract, sesame yeast is a plant-derived physiologically active material containing trace essential mineral organic selenium at a high concentration. In addition, it is a substance rich in essential minerals such as potassium, magnesium, zinc, manganese, and copper, and antioxidant lignans unique to sesame (sesamin and sesamolin).

Glutathione yeast contains glutamic acid, cysteine, and glycine. The glutathione yeast has an antioxidant action of glutathione and has an effect of contributing to improving blood pressure. Glutathione yeast is preferably contained in an amount of 0.5 to 0.9 wt%.

The pine bark dry extract, the fermented sesame dry extract, and glutathione yeast are formed using an excipient containing starch, lactose, and the like as raw materials. This is because the powder becomes uniform, the tablet is solidified, and quality degradation due to moisture absorption is prevented. The form thereof is a tablet, a capsule, or a powder. Since it is a therapeutic and prophylactic agent to be administered to small animals, a tablet form is preferable. The powder is difficult to be administered to small animals, but there is a method in which the powder is mixed with feed and administered.

### <Mixing Ratio>

In the blending example of the atrial natriuretic peptide lowering, therapeutic, and prophylactic agent of the present invention, the numerical values as shown in the table in Fig. 1 were optimal. The pine bark dry extract is 5 to 9 wt%, the fermented sesame dry extract is 5 to 9 wt%, and the excipient is 79 to 87 wt%.

When the amount of the atrial natriuretic peptide lowering, therapeutic, and prophylactic agent to be administered to a small or medium-sized dog having a body weight of about 10 kg is set to 140 mg, 7 to 13 mg of the fermented sesame dry extract, 7 to 13 mg of the fermented sesame dry extract, 0.7 to 1.3 mg of glutathione yeast, and 110 to 122 mg of the excipient are suitable.

### <Supplement>

The atrial natriuretic peptide lowering, therapeutic, and prophylactic agent of the present invention can be blended with potassium, magnesium, zinc, manganese, or copper as an essential mineral supplement.

In addition, antioxidant lignans including sesamin and sesamolin can be blended with the atrial natriuretic peptide lowering, therapeutic, and prophylactic agent of the present invention as supplements.

### <Sugar-Coated Tablet in Which Double Sugar Coating is Formed>

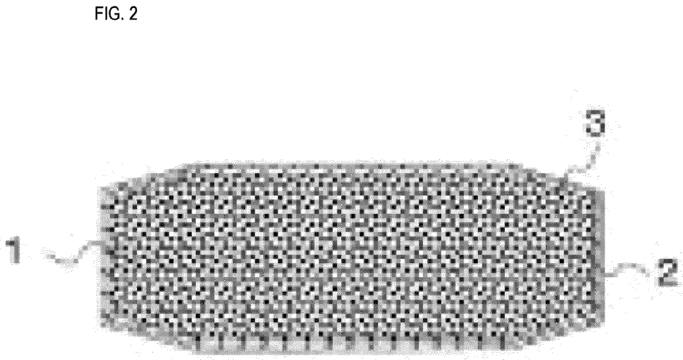

Fig. 2 is an enlarged cross-sectional view showing a sugar-coated tablet-type atrial natriuretic peptide lowering, therapeutic, and prophylactic agent in which a double sugar coating is formed.

When the atrial natriuretic peptide lowering, therapeutic, and prophylactic agent of the present invention is administered to a small animal, a sugar-coated tablet may adhere to the palate of the small animal, or may adhere to the throat or the inner wall of the esophagus. When the sugar of the sugar-coated tablet dissolves before reaching the stomach and the drug inside is exposed to the mouth and throat, the smell and taste of the drug become a concern, and the animal does not like administration of the drug. Therefore, the sugar of the sugar-coated tablet is doubly coated with a sugar in which an outer sugar coating is more water-soluble than an inner sugar coating to make it difficult to adhere to the inner wall of the throat and esophagus.

For example, polysaccharides such as starch, glycogen, and cellulose are used as an inner sugar coating 2 coated on a tablet 1. This is because the drug is protected from being dissolving until reaching the stomach. About 5 minutes after attachment of saliva and gastric juice, the sugar coating dissolves and the drug 1 inside is exposed.

As an outer sugar coating 3, monosaccharides such as glucose and fructose, and disaccharides such as sucrose and lactose, which are more water-soluble than the inner sugar coating 2, are used. It utilizes the property of being easily soluble in digestive enzymes of saliva. By forming the outer sugar coating 3 of the tablet with a material having a readily soluble property, it is possible to prevent the tablet 1 from adhering to the palate or adhering to the inner wall of the throat or esophagus.

### <Double Capsule>

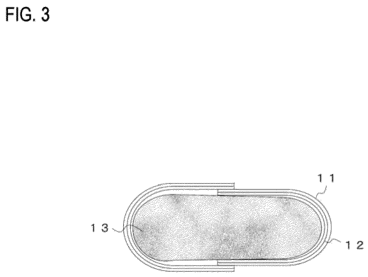

Fig. 3 is an enlarged cross-sectional view showing an atrial natriuretic peptide lowering, therapeutic, and prophylactic agent filled in a double capsule.

The double capsule also prevents the capsule from adhering to the palate of the small animal or adhering to the throat or the inner wall of the esophagus. This is because when gelatin and sugar in the capsule dissolve before the capsule reaches the stomach and the drug inside is exposed to the mouth and throat, the odor and taste of the drug become a concern and the animal does not like administration of the drug. Therefore, a capsule having a double structure in which an outer coating 11 is formed of a gelatinous material that is more water-soluble than an inner coating 12 (starch) is filled with a drug 13.

For example, the inner coating 12 of the capsule has a function of sufficiently drying vegetable fibers, starch, or the like to form a hard coating and protecting the drug 13 filled therein. About 5 minutes after attachment of saliva and gastric juice, the sugar coating dissolves and the drug 13 inside is exposed.

The outer coating 11 of the capsule is formed in a form that is more water-soluble than the inner coating 12. For example, gelatin has a property of being easily soluble in a digestive enzyme of saliva and a digestive enzyme of the stomach as compared with vegetable fibers, starch, or the like. By forming the outer side of the capsule with a material having a readily soluble property, it is possible to prevent the capsule from adhering to the palate or adhering to the inner wall of the throat or esophagus.

### [Example 2]

### <Method for Producing Atrial Natriuretic Peptide Lowering, Therapeutic, and Prophylactic Agent>

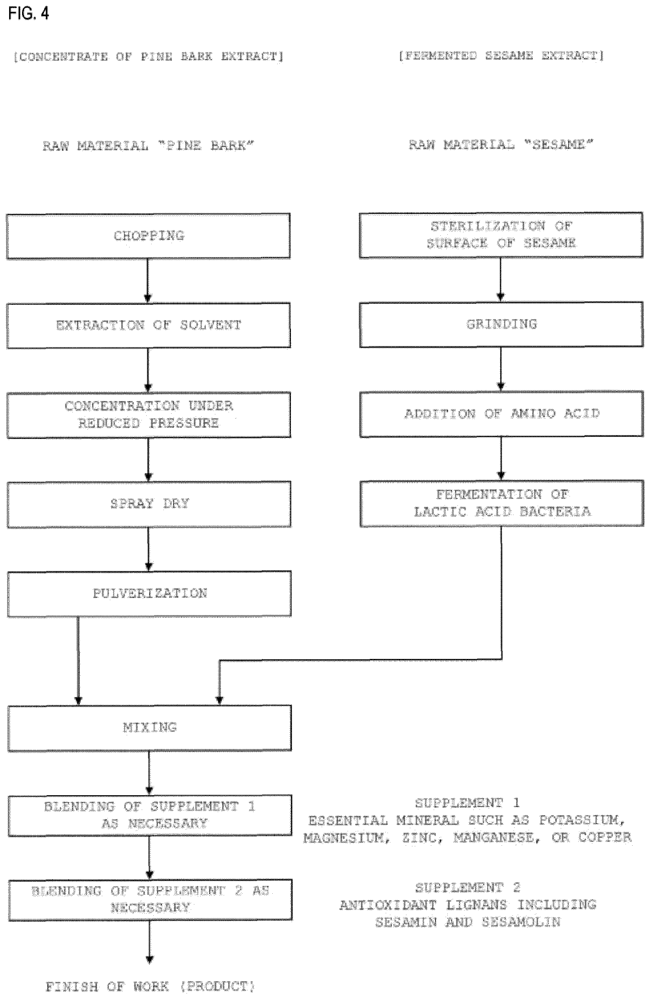

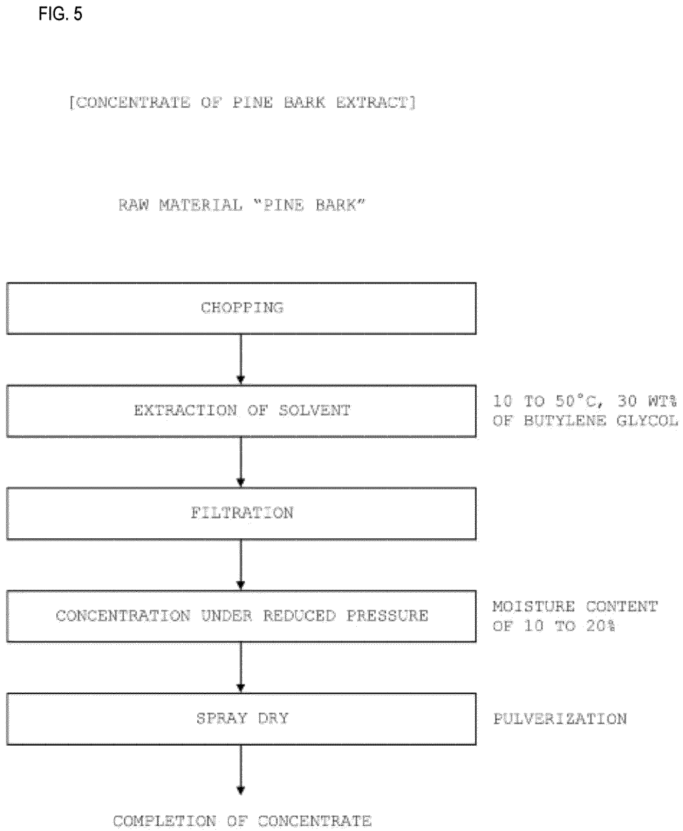

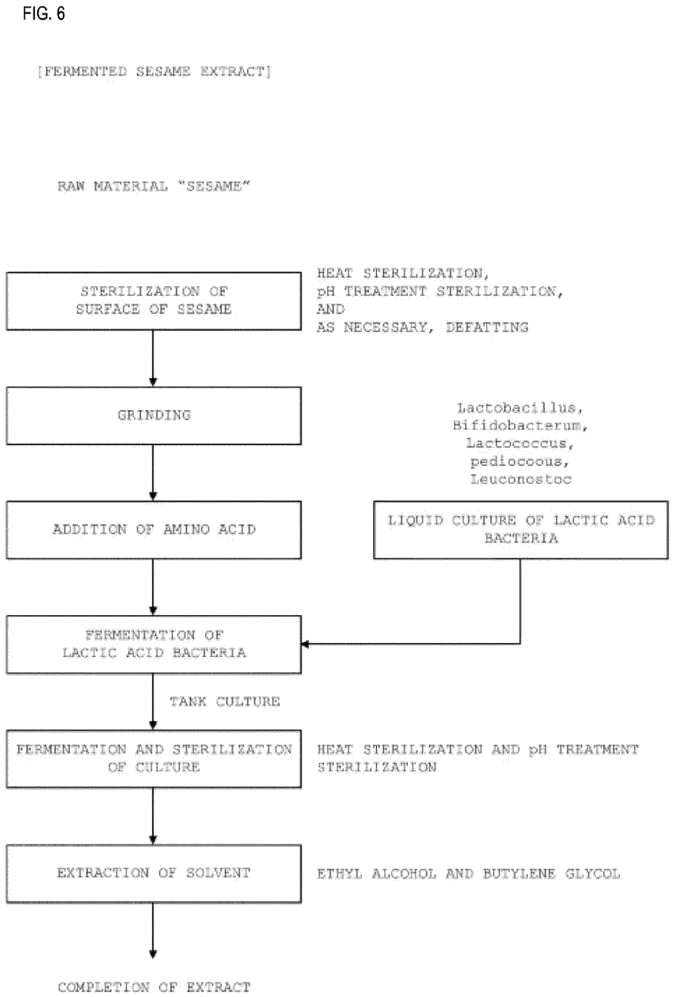

Fig. 4 is a flowchart showing a method for producing an atrial natriuretic peptide lowering, therapeutic, and prophylactic agent of the present invention in Example 2. Fig. 5 is a flowchart showing a process for producing a pine bark dry extract in detail. Fig. 6 is a flowchart showing a process for producing a fermented sesame dry extract in detail.

In a method for producing an atrial natriuretic peptide lowering, therapeutic, and prophylactic agent according to the present invention, bark of pine trees which have been grown under exposure to intense ultraviolet light on a coast is collected, and the bark is chopped and pulverized. The pulverized product is extracted using an extract of butylene glycol. For example, at 10 to 50°C, extraction is performed using 30 wt% or more of a butylene glycol extract. Note that the solvent is not limited to butylene glycol, and a solvent (oil agent) such as ethanol can be used.

The extract of the pine bark is filtered and concentrated under reduced pressure. The pine bark extract is concentrated, for example, to a moisture content of 10 to 30%.

Thereafter, the concentrated extract is spray-dried and pulverized to obtain a pine bark dry extract.

Regarding the fermented sesame dry extract, an amino acid is added to sesame, and one or more bacteria selected from lactic acid bacteria are inoculated and fermented to produce a fermented sesame dry extract. In order to efficiently ferment sesame, it is necessary to sterilize various bacteria on the surface of sesame. The sterilization method includes heat sterilization and pH treatment sterilization, and defatting may be performed as necessary.

Sesame is fermented in a granular state, and finely ground sesame is used for better fermentation.

The fermentation of sesame is carried out in the presence of water. Examples of the fermentation method include a tank culture in which fermentation is performed by adding a large amount of water, a solid culture in which fermentation is performed by adding a small amount of water, and a method in which fermentation is performed in the form of an intermediate paste. It is more preferable to use a tank culture method capable of processing a large volume relatively quickly.

Examples of the type of lactic acid bacteria to be inoculated include the genus Lactobacillus, the genus Bifidobacterium, the genus Lactococcus, the genus Pediococcus, and the genus Leuconostoc, and among them, a strain that does not produce a toxic agent can be used. In particular, Lactobacillus casei is preferable because it efficiently ferments the substrate in sesame and efficiently converts sesaminol to sesamol, and Lactobacillus reuteri is more preferable because it efficiently produces γ-aminobutyric acid.

As the amount of lactic acid bacteria to be inoculated, a liquid or solid containing sesame is inoculated with 0.5 to 10 wt% of a bacterial solution cultured in a liquid, and is particularly preferably inoculated with 1.0 wt% of the bacterial solution under aseptic conditions.

In the fermented sesame dry extract thus produced, a molecular weight of sesame flakes was reduced by fermentation to remove unnecessary fiber components, and selenium and other essential mineral components were highly concentrated by a separation and purification method. This is a pure plant-derived physiologically active material (phytochemical) powder containing a trace amount of essential mineral organic selenium derived from sesame at a high concentration.

The property of the fermented sesame dry extract is a light brown powder, and selenium is contained in an amount of 75 ppm or more (average value of 100 ppm). An oil content is 20% or less, a moisture content is 8% or less, arsenic (as As₂O₃) is 1 ppm or less, heavy metals (as Pb) is 10 ppm or less, the number of viable bacteria is 3 × 10³ cells/g or less, and E. coli is negative.

The fermented sesame dry extract is blended with the concentrated pine bark dry extract so that a mixing ratio of the pine bark dry extract and the fermented sesame dry extract is 0.5 to 2.0 of a weight ratio of the fermented sesame dry extract with respect to 1 of a weight ratio of the pine bark dry extract, such that the production of the atrial natriuretic peptide lowering, therapeutic, and prophylactic agent is completed.

Thereafter, a product of the atrial natriuretic peptide lowering, therapeutic, and prophylactic agent is produced in the form of a tablet, a capsule, or the like.

### <Administration of Atrial Natriuretic Peptide Lowering, Therapeutic, and Prophylactic Agent>

As a standard amount of the atrial natriuretic peptide lowering, therapeutic, and prophylactic agent of the present invention to be administered to a small animal such as a dog, for example, when the weight of the dog is 10 kg or less, about 560 mg is preferable.

### <Description of Cases>

The effect of the atrial natriuretic peptide lowering, therapeutic, and prophylactic agent of the present invention given to animals (dogs) suffering from mitral regurgitation (MR) will be described.

In the results of administering the atrial natriuretic peptide lowering, therapeutic, and prophylactic agent of the present invention to cases of 26 dogs with mitral regurgitation (MR) for 1 month, changes in atrial natriuretic peptide (ANP) values are shown in Table 1. The ANP value is a hormone mainly secreted from the atrium, and is a numerical value indicating an important role in body fluid volume or blood pressure adjustment. After administration, the numerical value often decreases. Single administration was performed in 3 cases (3 dogs), and combination use with a drug was performed in 23 cases (23 dogs).

**[Table 1]**

| Table 1 Changes in ANP value | | |
|---|---|---|
| * Case in which ANP value is less than 10 is classified as no change | | |
| Decrease | **10 cases** | **40%** |
| No change | **10 cases** | **40%** |
| Increase | **6 cases** | **20%** |

### <Changes in ANP Value in 26 Dogs>

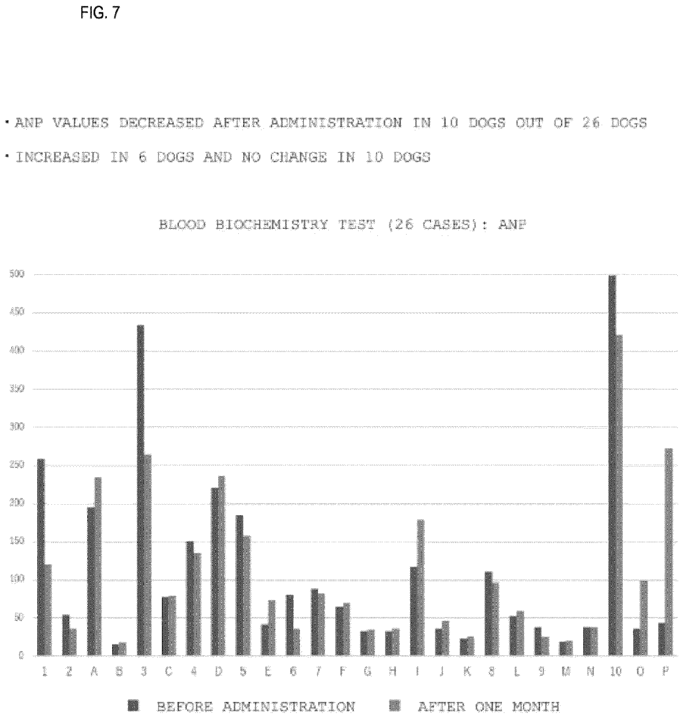

Fig. 7 is a graph showing changes in ANP value before and after the atrial natriuretic peptide lowering, therapeutic, and prophylactic agent of the present invention is given to animals (dogs) suffering from mitral regurgitation (MR).

Fig. 7 also shows changes in the ANP value. The left of each bar line is a numerical value before administration, and the right is a numerical value for 1 month of administration. It is shown that the numerical value of the ANP value after administration decreased in 10 dogs out of 26 dogs. In addition, the ANP value increased in 6 dogs and did not change in 10 dogs.

Table 2 shows changes in quality of life (QOL) value. The QOL value is a numerical value obtained by quantifying the effect of the disease or treatment on the subjective sense of health (mental health, vitality, pain, and the like) of a patient (dog) and daily activities. As shown in Table 2, most numerical values are improved after administration.

**[Table 2]**

| Table 2 Changes in QOL | | |
|---|---|---|
| Improvement | **15 cases** | **57.7%** |
| No change | **10 cases** | **38.5%** |
| Worsening | **1 cases** | **3.8%** |

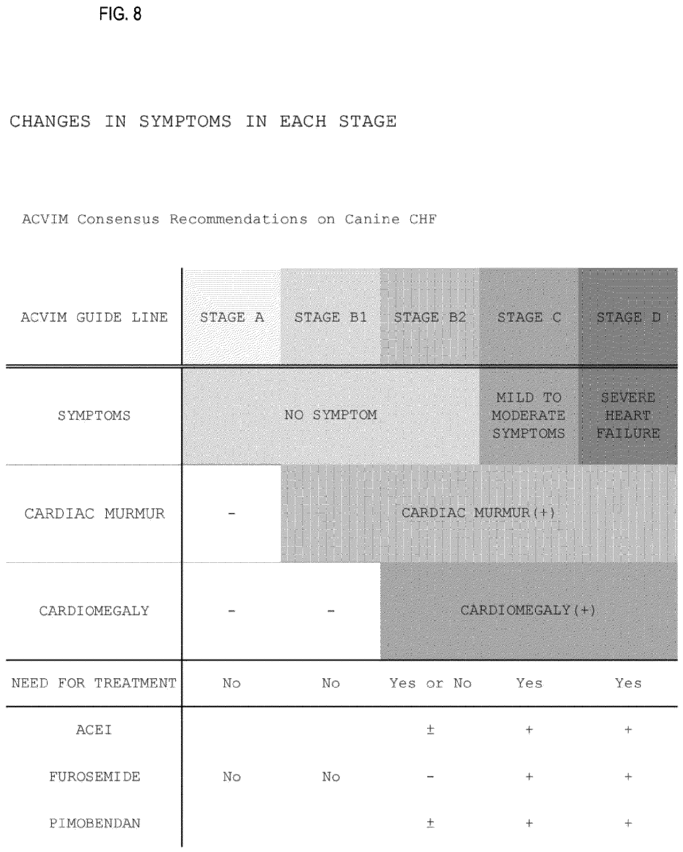

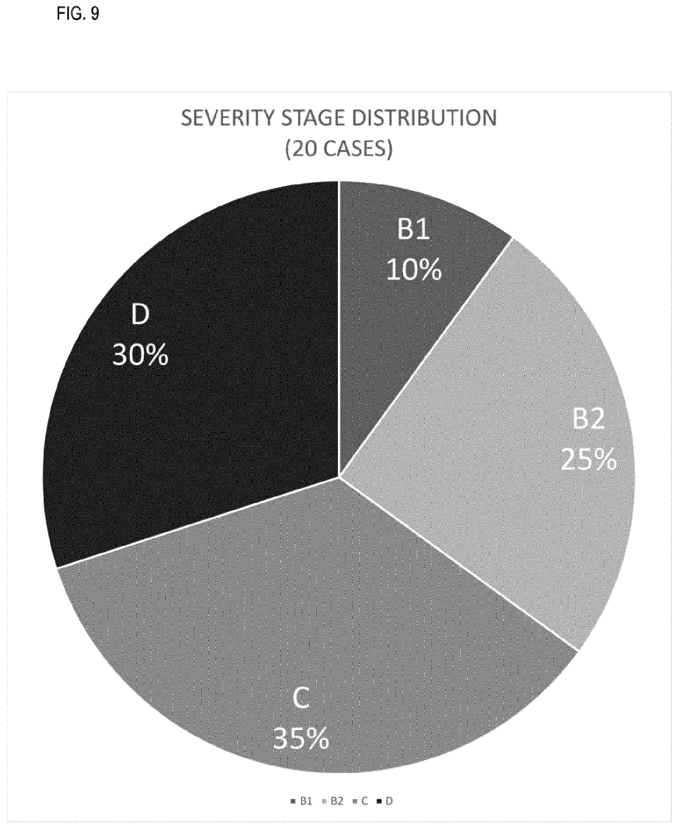

Fig. 8 is a graph showing changes in symptoms in each stage. Fig. 9 is a graph showing a severity stage distribution.

Fig. 8 explains "symptoms", "cardiac murmur", "cardiomegaly", "need for treatment", and the need for administration of "ACEI", "furosemide", and "pimobendan" in the stages from Stage A to Stage D. Fig. 9 is a graph showing the severity stage distribution of 20 cases from Stage B1 to Stage D.

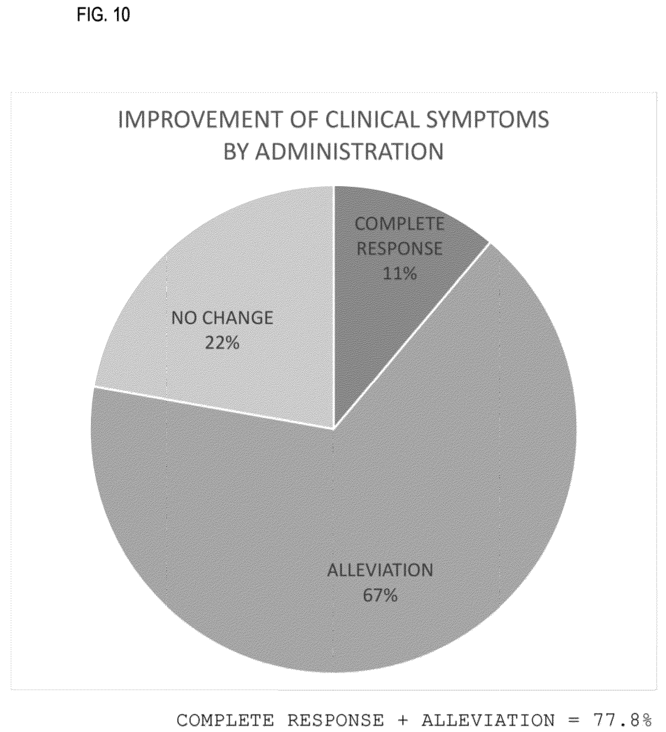

Fig. 10 is a graph showing improvement of clinical symptoms when the atrial natriuretic peptide lowering, therapeutic, and prophylactic agent is administered to 9 dogs in Group A.

Dos diagnosed with mitral regurgitation were observed for 1 month. The dogs were divided into two groups according to the examination contents of the veterinarian. Group A includes 9 cases (dogs) in which the ANP value is measured. Group B was performed focusing on clinical follow-up. 12 cases (dogs) are included. In this observation, the owner of each dog observed a breathing rate monitor at bedtime. Similarly, the state during coughing or taking a walk was observed. Not that the contents of the atrial natriuretic peptide lowering, therapeutic, and prophylactic agent of the present invention for the administration period (1 month) are not changed. In addition, there was no concomitant use of other supplements.

The improvement of clinical symptoms by administration of the atrial natriuretic peptide lowering, therapeutic, and prophylactic agent of the present invention will be described. For the 9 dogs in Group A, the improvement after administration shows a change in "complete response" which is significantly effective, "alleviation" in which the symptoms are relieved, "no change", or "worsening". Table 3 is a table showing improvement of clinical symptoms when the atrial natriuretic peptide lowering, therapeutic, and prophylactic agent is administered to 9 dogs in Group A. The complete response and the alleviation were 77.8%.

**[Table 3]**

| Table 3 Clinical symptoms by admini | | | | |
|---|---|---|---|---|
| Group A: 9 cases | | | | |
| Performing ANP measurement | | | | |
| | Complete response | Alleviation | No change | Worsening |
| Number of dogs (9 dogs in total) | **1 cases** | **6 cases** | **2 cases** | **0 cases** |
| Improvement and alleviation rate | **11.1%** | **66.7%** | **22.2%** | **0.0%** |

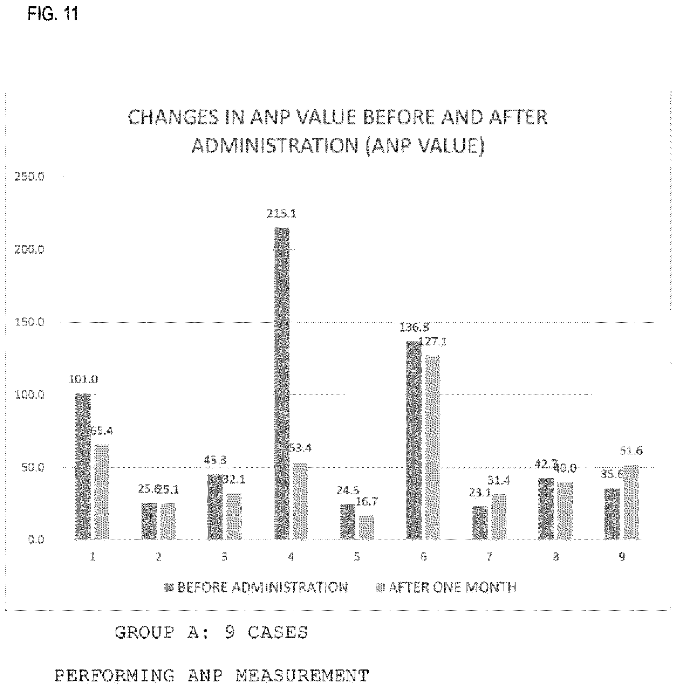

Fig. 11 is a graph showing improvement of clinical symptoms by administration of the atrial natriuretic peptide lowering, therapeutic, and prophylactic agent of the present invention in 9 cases of Group A.

Each two bars indicate the ANP values of the dog in each case, the dark gray bar on the left is the ANP value before administration of the atrial natriuretic peptide lowering, therapeutic, and prophylactic agent of the present invention, and the light gray bar on the right is the ANP value 1 month after administration. After administration, the numerical value often decreases.

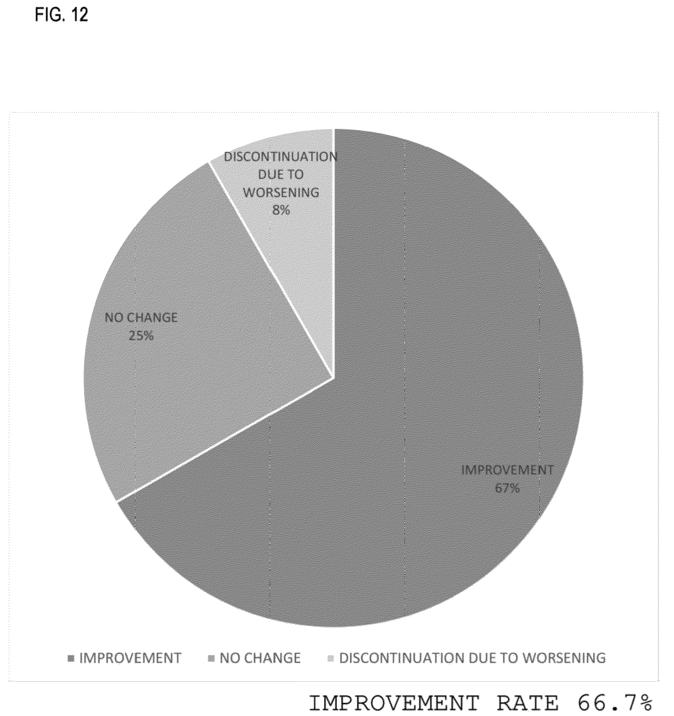

Fig. 12 is a graph showing improvement of clinical symptoms by administration of the atrial natriuretic peptide lowering, therapeutic, and prophylactic agent of the present invention in 12 cases of Group B.

Next, changes in "improvement", "no change", or "discontinuation due to worsening" after administration are shown for 12 dogs in Group B. Table 4 is a table for improvement of clinical symptoms by administration of the atrial natriuretic peptide lowering, therapeutic, and prophylactic agent. The improvement rate was 66.7%.

**[Table 4]**

| Table 4 Clinical symptoms by administration | | | |
|---|---|---|---|
| Group B: 12 cases | | | |
| Focused on clinical follow-up | | | |
| | Improvement | No change | Discontinuation due to worsening |
| Number of dogs (12 dogs in total) | **8 cases** | **3 cases** | **1 cases** |
| Improvement and alleviation rate | **66.7%** | **25.0%** | **8.3%** |

Next, changes in resting respiratory rate will be described for both Group A and Group B. Table 5 is a table showing improvement of clinical symptoms by administration of the atrial natriuretic peptide lowering, therapeutic, and prophylactic agent of the present invention for 11 cases. Before and after administration, the numerical value often decreases after administration. That is, it indicates that respiration at rest is improved.

**[Table 5]**

| Table 5 Changes in resting respiratory rate | | |
|---|---|---|
| Group A + Group B = 11 | | |
| [times/min] | | |
| Cases | Before administration | After administration |
| 1 | **43** | **35** |
| 2 | **19** | **15** |
| 3 | **16** | **11** |
| 4 | **13** | **12** |
| 5 | **19** | **10** |
| 6 | **64** | **62** |
| 7 | **23** | **26** |
| 8 | **18** | **13** |
| 9 | **18** | **12** |
| 10 | **42** | **36** |
| 11 | **15** | **12** |
| Average | **26.36** | **22.18** |

| | | |
|---|---|---|
| Count at bedtime | | |

### <Comparison of X-Ray Images of Cases>

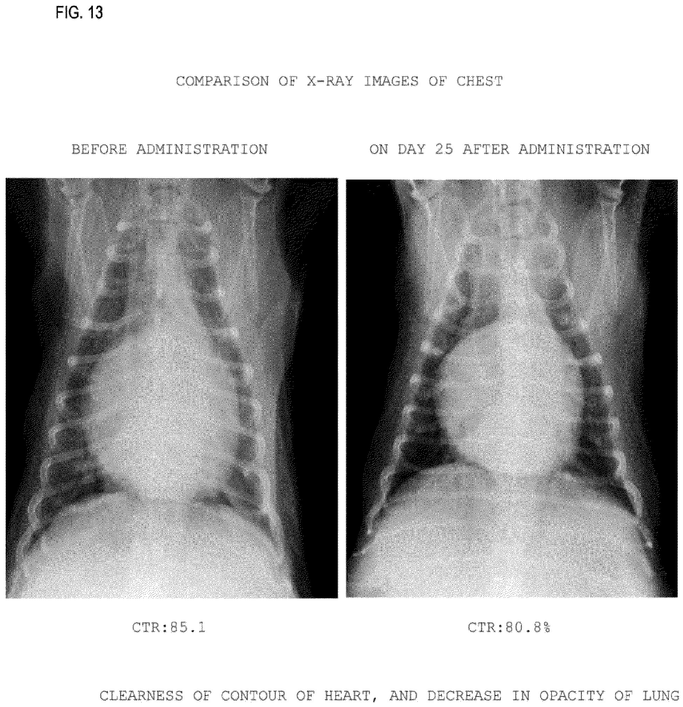

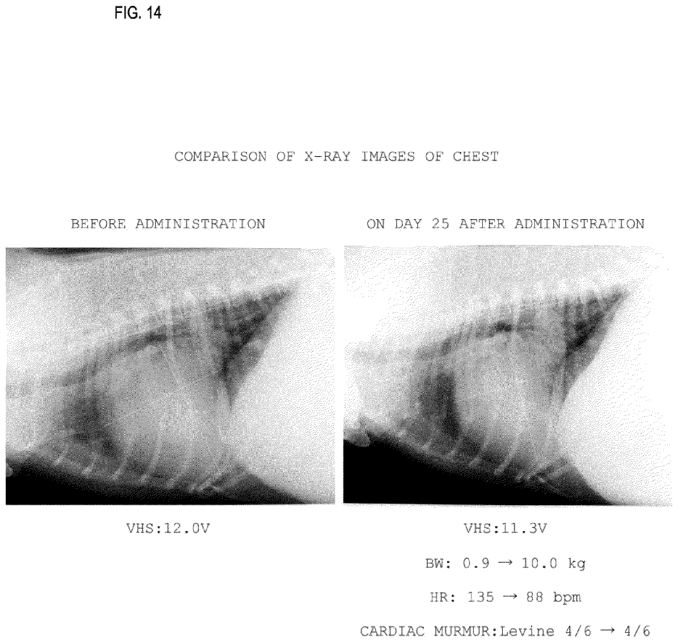

Figs. 13 and 14 are images showing a comparison of X-ray images for each case.

Fig. 13 shows X-ray images of the chest of a dog, and the left of the drawing is an image before administration of the atrial natriuretic peptide lowering, therapeutic, and prophylactic agent of the present invention. The right is an image on Day 25 after administration. These images show that the contour of the heart was clearly observed and the opacity of the lung was improved by administration of the atrial natriuretic peptide lowering, therapeutic, and prophylactic agent of the present invention.

Fig. 14 shows X-ray images of the chest of the dog captured from another angle, and the left of the drawing is an image before administration of the atrial natriuretic peptide lowering, therapeutic, and prophylactic agent of the present invention. The right is an image on Day 25 after administration. These images show that the contour of the heart was clearly observed and the opacity of the lung was improved by administration of the atrial natriuretic peptide lowering, therapeutic, and prophylactic agent of the present invention.

### <Comparison of Ultrasonography (Echography) Images of Cases>

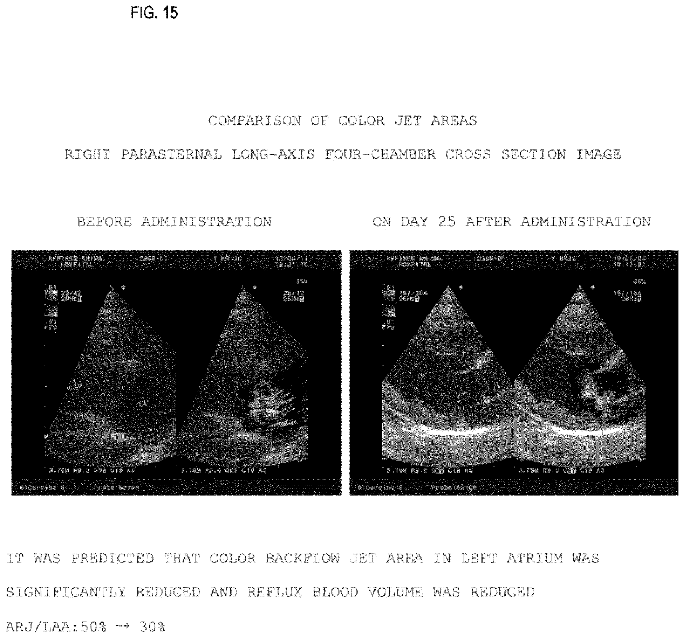

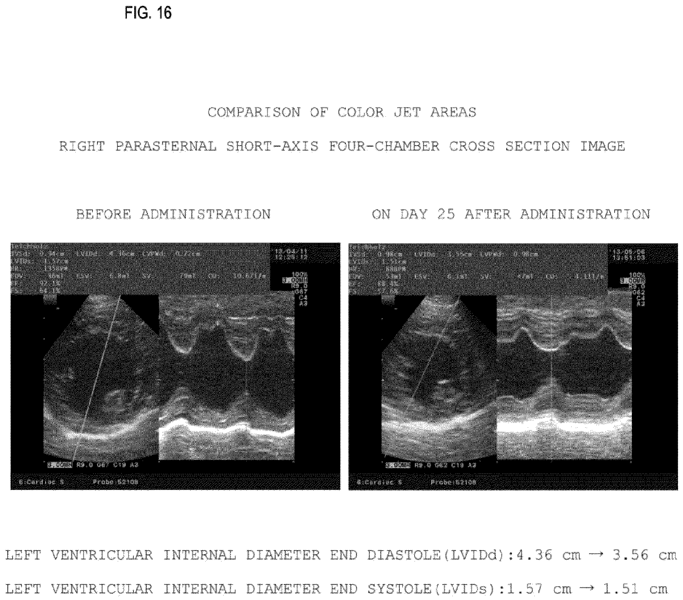

Figs. 15 and 16 are comparisons of color jet areas, and are images showing comparisons of ultrasonography (echography) for the right parasternal four-chamber cross sections.

Fig. 15 is an image of ultrasonography (echography) of the right parasternal long-axis of the dog, and the left of the drawing is an image before administration of the atrial natriuretic peptide lowering, therapeutic, and prophylactic agent of the present invention. The right is an image on Day 25 after administration. From these images, it was predicted that the color backflow jet area in the left atrium was significantly reduced and the reflux blood volume was reduced by the administration of the atrial natriuretic peptide lowering, therapeutic, and prophylactic agent of the present invention. ARJ/LAA decreased from 50% to 30%.

Fig. 16 is an image of ultrasonography (echography) of the right parasternal short-axis of the dog, and the left of the drawing is an image before administration of the atrial natriuretic peptide lowering, therapeutic, and prophylactic agent of the present invention. The right is an image on Day 25 after administration. The left ventricular internal diameter end diastole (LVIDd) changed from 4.36 cm to 3.56 cm.

The left ventricular internal diameter end systole (LVIDs) changed from 1.57 cm to 1.51 cm.

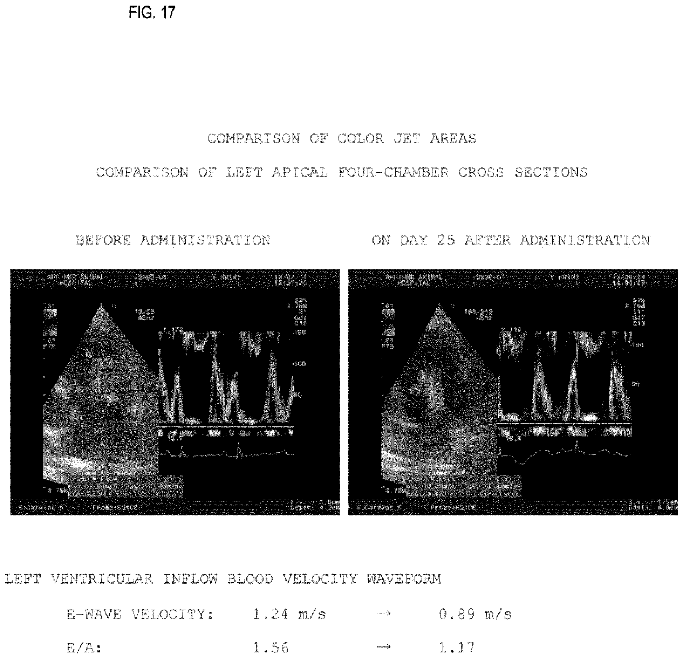

Fig. 17 shows a comparison of color jet areas, and is an image showing a comparison of ultrasonography (echography) for a left ventricular inflow blood velocity waveform.

Fig. 17 is an image of ultrasonography (echography) of the left ventricular inflow blood velocity waveform of the dog, and the left of the drawing is an image before administration of the atrial natriuretic peptide lowering, therapeutic, and prophylactic agent of the present invention. The right is an image on Day 25 after administration. The E-wave velocity (diastolic early wave) changed from 1.24 m/s to 0.89 m/s. E/A changed from 1.56 to 1.17. The A-wave velocity is an atrial systolic wave.

### <Sick Sinus Syndrome>

A case of Miniature Schnauzer, spay female, 8 years old, and a body weight of 7.1 kg will be described. The case has diabetes and Cushing's syndrome as previous diseases. The case has mitral regurgitation (III/VI). Clinical symptoms are symptoms of syncope at rest due to sinus bradycardia (ISACHC classification Ib ACVIM classification B2). The concomitant drug is vetmedin, cilostazol, and herbal medicine (fructus edulis/plant extract).

Sick sinus syndrome is a disease in which bradycardia occurs when an abnormality occurs in cells of the sinoatrial node giving a command of contraction of the heart.

Sick sinus syndrome is a combination of sinus bradycardia (the patient feels that the heart is beating slowly, causing subjective symptoms such as wobble, dizziness, and a floating sensation), sinus stop (there are many dizziness and wobbles, and syncope occurs when the sinus stop time is long (Adams-Stokes seizure)), sinoatrial block (a condition in which cells of the atrioventricular node that transmit the command for cardiac contraction are impaired and the command is not properly transmitted from the atrium to the ventricle), and supraventricular tachycardia (in a condition in which the (paroxysmal) pulse becomes faster for some reason and continues (tachycardia), and the pulse that is faster coming from the atrium and its vicinity (supraventricular) is paroxysmal supraventricular tachycardia), and is thus not a single disease. Characteristics of the sick sinus syndrome include (A) severe sinus bradycardia, (B) advanced sinus stop, and (B) bradycardia/tachycardia syndrome.

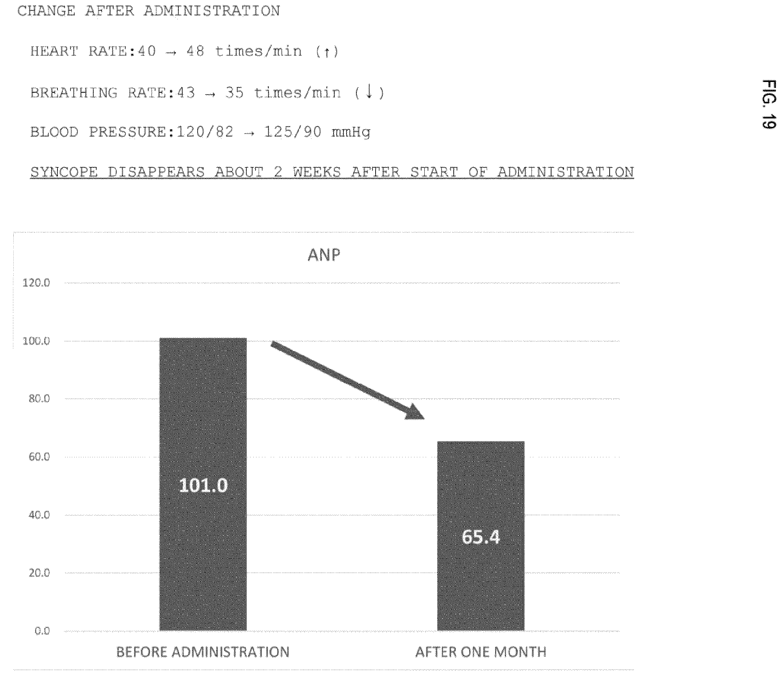

Fig. 19 shows the examination of cardiac motion of a dog having symptoms of bradycardia and tachycardia syndrome. Fig. 19 is an explanatory view showing improvement of clinical symptoms when the atrial natriuretic peptide lowering, therapeutic, and prophylactic agent of the present invention is administered to a dog having symptoms of bradycardia and tachycardia syndrome.

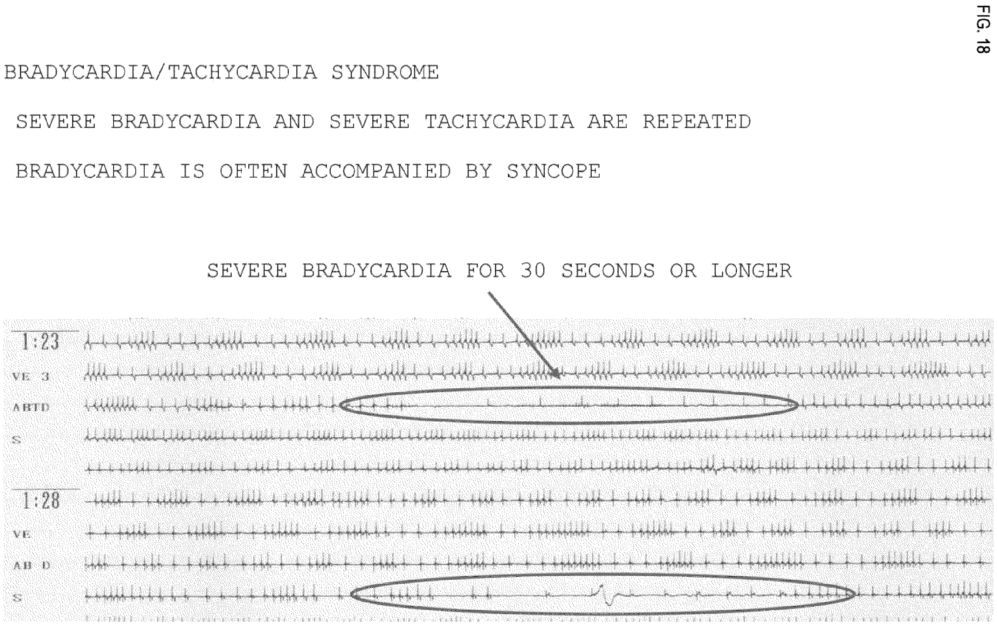

Dogs with bradycardia/tachycardia syndrome repeat severe bradycardia and severe tachycardia. In addition, bradycardia is often accompanied by syncope. In Fig. 18, a portion marked with an oval shape indicates a state in which severe bradycardia for 30 seconds or longer appears.

When the atrial natriuretic peptide lowering, therapeutic, and prophylactic agent of the present invention was administered to the dog, the heart rate was increased from 40 to 48 times/min as shown in Fig. 19. The breathing rate dropped from 43 to 35 times/min. The blood pressure increased from 120/82 to 125/90 mmHg. Furthermore, syncope disappeared about 2 weeks after the start of administration.

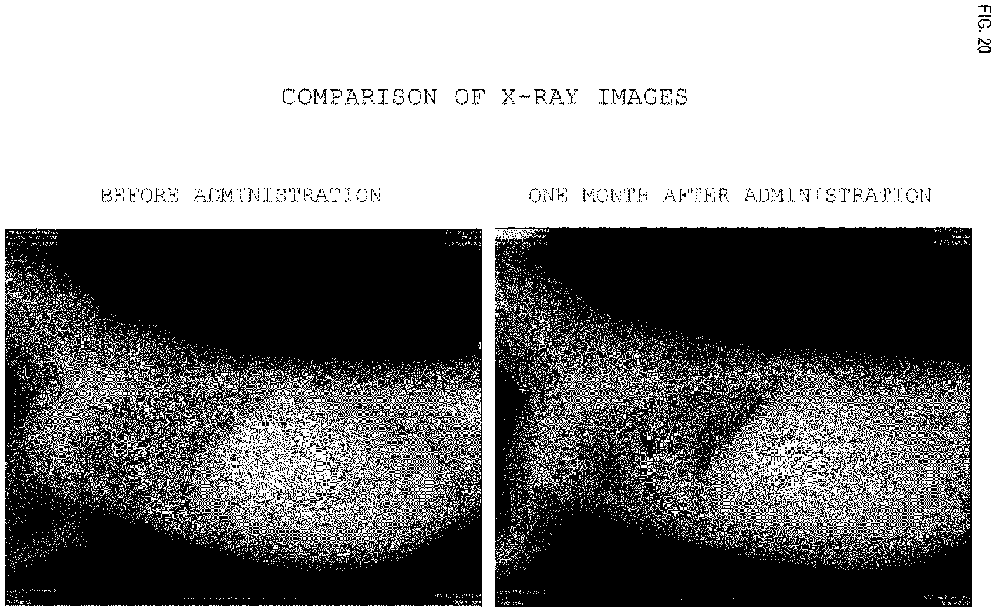

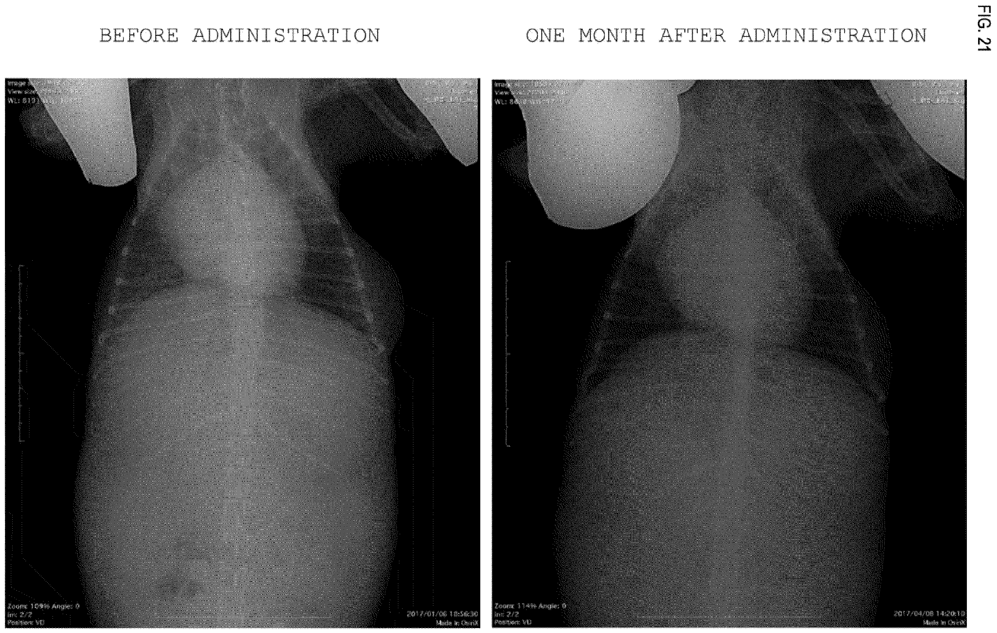

Figs. 20 and 21 are images showing comparisons of X-ray images when the atrial natriuretic peptide lowering, therapeutic, and prophylactic agent of the present invention is administered to a dog having symptoms of a sick sinus syndrome.

The left of the drawing is an image before administration of the atrial natriuretic peptide lowering, therapeutic, and prophylactic agent of the present invention. The right of the drawing is an image 1 month after administration.

The atrial natriuretic peptide lowering, therapeutic, and prophylactic agent of the present invention can promote NO synthesis. Prostacyclin production can be promoted. Endothelin production can be suppressed. As a result, it contributes to improving pulmonary edema and disappearance of bradycardia syncope.

Note that the present invention is not limited to the above-described embodiment of the invention as long as it has a configuration in which an agent can be taken at all times like a supplement without impairing the health of small animals, and can more effectively suppress heart disease such as myxomatous mitral valve disease by using a naturally occurring substance, and various modifications can be made without departing from the gist of the present invention.

### Industrial Applicability

The atrial natriuretic peptide lowering, therapeutic, and prophylactic agent of the present invention can be used for a health supplement food that works effectively when given to a small animal suffering from mitral regurgitation, particularly a dog.

### Reference Signs List

- 1: Tablet (drug)
- 2: Inner sugar coating
- 3: Outer sugar coating
- 11: Outer coating
- 12: Inner coating
- 13: Drug

## Claims

1. An atrial natriuretic peptide lowering, therapeutic, and prophylactic agent for an animal suffering from mitral regurgitation,
wherein the atrial natriuretic peptide lowering, therapeutic, and prophylactic agent is obtained by:
extracting bark of pine trees at 10 to 50°C using 30 wt% or more of an extract to produce a pine bark extract, the pine trees having been grown on the southwestern coast of France under exposure to intense ultraviolet light; concentrating the pine bark extract to a moisture content of 5 to 20%;
adding 30 wt% or more of an amino acid to sesame seeds, inoculating one or more bacteria selected from lactic acid bacteria, and performing fermentation at 10 to 50°C to produce a fermented sesame dry extract; and
blending the fermented sesame dry extract with the concentrated pine bark dry extract so that a weight mixing ratio of the pine bark dry extract and the fermented sesame dry extract is 0.5 to 2.0 of a weight ratio of the fermented sesame dry extract with respect to 1 of a weight ratio of the pine bark dry extract.

2. The atrial natriuretic peptide lowering, therapeutic, and prophylactic agent according to claim 1, wherein the atrial natriuretic peptide lowering, therapeutic, and prophylactic agent further contains 0.5 to 0.9 wt% of glutathione yeast containing glutamic acid, cysteine, and glycine.

3. The atrial natriuretic peptide lowering, therapeutic, and prophylactic agent according to claim 1, wherein the fermented sesame dry extract contains organic selenium in a weight ratio of 75 ppm or more.

4. The atrial natriuretic peptide lowering,
therapeutic, and prophylactic agent according to claim 1, wherein the atrial natriuretic peptide lowering, therapeutic, and prophylactic agent is obtained by blending potassium, magnesium, zinc, manganese, or copper as an essential mineral supplement.

5. The atrial natriuretic peptide lowering,
therapeutic, and prophylactic agent according to claim 1, wherein the atrial natriuretic peptide lowering, therapeutic, and prophylactic agent is obtained by blending antioxidant lignans including sesamin and sesamolin.

6. The atrial natriuretic peptide lowering, therapeutic, and prophylactic agent according to claim 1, wherein the atrial natriuretic peptide lowering, therapeutic, and prophylactic agent is obtained by coating a tablet solidified to a certain hardness by pressure with polysaccharides including starch, glycogen, and cellulose and coating the outside of the table with water-soluble monosaccharides and disaccharides including starch and dextrin.

7. The atrial natriuretic peptide lowering, therapeutic, and prophylactic agent according to claim 1, wherein the atrial natriuretic peptide lowering, therapeutic, and prophylactic agent is filled in a capsule having a double structure in which an inner coating (12) is formed of starch and an outer coating (11) is formed of a gelatinous material that is more water-soluble than the inner coating (12).

8. A method for producing an atrial natriuretic peptide lowering, therapeutic, and prophylactic agent, the method comprising:
extracting bark of pine trees at 10 to 50°C using 30 wt% or more of an extract to produce a pine bark extract, the pine trees having been grown on the southwestern coast of France under exposure to intense ultraviolet light;
concentrating the pine bark extract to a moisture content of 5 to 20%;
adding 30 wt% or more of an amino acid to sesame seeds, inoculating one or more bacteria selected from lactic acid bacteria, and performing fermentation at 10 to 50°C to produce a fermented sesame dry extract; and
blending the fermented sesame dry extract with the concentrated pine bark dry extract so that a weight mixing ratio of the pine bark dry extract and the fermented sesame dry extract is 0.5 to 2.0 of a weight ratio of the fermented sesame dry extract with respect to 1 of a weight ratio of the pine bark dry extract.
